# EUROPEAN PATENT APPLICATION

(11) **EP 1 064 937 A1**
(43) Date of publication of application: **03.01.2001**
(21) Application number: 99401605.3
(22) Date of filing: 28.06.1999
(51) Int. Cl.: A61K 9/50

(54) **Timed dual release dosage forms comprising a short acting hypnotic or a salt thereof**

(71) Applicant: Sanofi-Synthélabo, 75013 Paris (FR)
(72) Inventor: Alaux, Gérard, 78650 Reynes (FR); Andre, Frédéric, 92160 Antony (FR); Ducassou, Jean, 64600 Anglet (FR); Lewis, Gareth, 91410 Dourdan (FR)
(74) Representative: Thouret-Lemaitre, Elisabeth

(57) **Abstract**

The present invention relates to timed dual release dosage forms of short acting hypnotics or salts thereof adapted to release the short acting hypnotic over a predetermined time period, according to a profile of dissolution characterised in that it comprises two release pulses, the first being immediate (lasting up to 30 minutes) and the second being delayed by a fixed time (this fixed time being between 50 and 200 minutes).

## Description

The present invention relates to timed dual release dosage forms comprising short acting hypnotics or salts thereof.

The short acting hypnotics can belong to all therapeutic classes:
- compounds of the therapeutic class of pyrazolopyrimidines, such as zaleplon,
- compounds of the therapeutic class of cyclopyrrolones, such as zopiclone and its enantiomers like (*R*)-zopiclone,
- compounds of the therapeutic class of benzodiazepines, such as triazolam, temazepam or brotizolam,
- compounds of the therapeutic class of phenothiazines, such as alimemazine or the tartrate thereof,
- compounds of the therapeutic class of imidazopyridines, such as zolpidem.
One of the preferred salts of zolpidem is zolpidem hemitartrate.

Up to now, according to the rapidity of action of this kind of active substances, only immediate release dosage forms were developed, which disintegrate rapidly in the gastrointestinal tract, dissolve in the fluid of the gastrointestinal tract and undergo systemic absorption, where the short acting hypnotic, which we will call hereinafter "the drug", can exert its pharmacological effect and induce sleep of the patient.

The new dosage forms according to the present invention enable first of all a sufficient blood level of short acting hypnotic to be obtained rapidly after administration in order to induce sleep, and then a second pulse of short acting hypnotic to be released after a fixed time after administration in order to maintain sleep.

Therefore, as a first object, the present invention provides timed dual release dosage forms comprising short acting hypnotics or salts thereof adapted to release over a predetermined time period, according to a profile of dissolution, characterized in that it comprises two release pulses, the first being immediate and the second being delayed to a fixed time.

The "total amount of drug" means the quantity by weight of the drug comprised in the whole dosage form according to the invention.

The immediate release portion of the profile (initial pulse) is defined as that proportion of the drug dissolved in 30 minutes in a suitable *in vitro* dissolution test. A suitable dissolution test is for example one of the methods described in example 1: method where measurement is carried out with the rotating paddle apparatus of the European pharmacopoeia, at a stirring speed of 50 rpm, in an aqueous buffer at pH between 1 and 7.5 at 37°C, or variations on this as well known to one skilled in the art.
The proportion of the drug dissolved during this pulse is the proportion of the total amount of the drug which is dissolved at 30 minutes. In an advantageous embodiment of the dosage forms according to the present invention 90% or more of that part of the drug allotted for the initial pulse is dissolved in 20 minutes and more preferably in 15 minutes. This embodiment is particularly advantageous for dosage forms comprising zolpidem or a salt thereof.

The delayed release portion of the profile is the part of the dissolution occurring after 30 minutes, measured in a suitable *in vitro* dissolution test, such as described in example 1.
The delayed release portion of the profile is defined by the percentage released at times T₁ and T₂, defined as follows.
T₁ describes the beginning of the second, delayed release pulse, and is defined as the time for release of 10% of the drug allotted for the delayed release portion of the profile.
T₂ describes the end of the delayed release pulse, and is defined as the time for release of 85% of the drug allotted for the delayed release portion of the profile.

The release of the delayed release pulse may be less rapid than the immediate release pulse. For example, the period (T₂-T₁) can last between 30 and 200 minutes.
Moreover, the delayed release pulse can begin between 50 minutes and 200 minutes after the beginning of dissolution, and preferably between 60 and 150 minutes, this range of time being defined as the "fixed time".

Indeed, the delayed release should be completed at a time after administration compatible with the desired time of sleep, and the time needed for elimination of the drug from the human body to a sufficiently low level roughly 8 hours after administration. In view of this, T₂ is between 2 and 6 hours and preferably between 2.5 and 5 hours.

The immediate release pulse can liberate between 40 to 70% of the total amount of the drug.

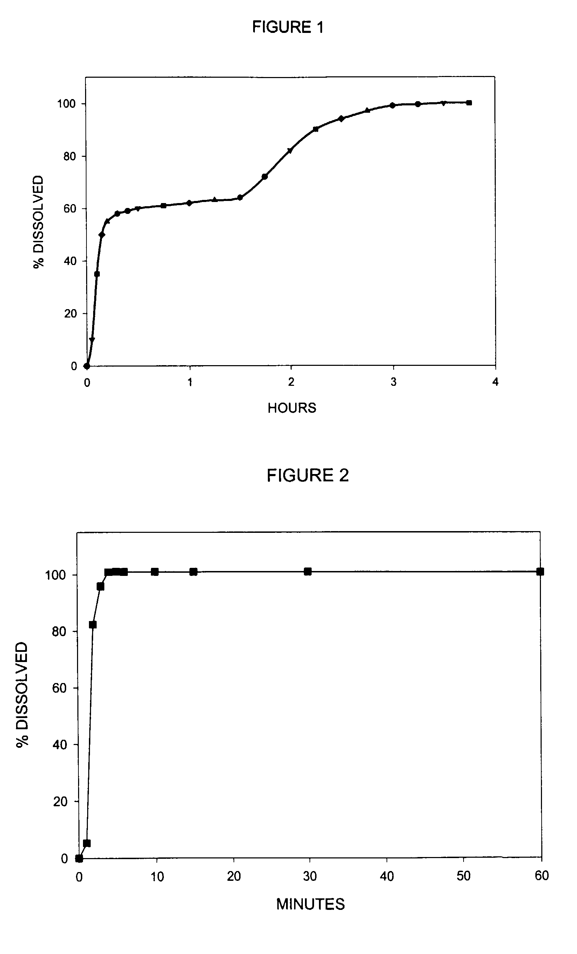

An example of such an *in vitro* release profile is given in figure 1, where 60% of the total amount of drug is released during the immediate release pulse, and the second, delayed release pulse occurs after 90 minutes (T₁) with a T₂ time being equal to 150 minutes.

As a second object, the present invention provides timed dual release dosage forms of short acting hypnotics or salts thereof, characterized in that they comprise two kinds of pharmaceutical entities of drug: one immediate release entity and one delayed release entity. The drug dissolved during the initial, immediate release pulse (before 30 minutes) is contained within the immediate release entity, and that liberated in the second, delayed release pulse (beginning after the fixed time) is contained within the delayed release entity.

Small quantities of the drug in a formulation for rapid release can be retained in the formulation and thus may be released at a time after 30 minutes from the beginning of the dissolution, and are thus included in the delayed release part of the profile. Similarly, small quantities of the drug incorporated in the delayed release pharmaceutical entity may be released before 30 minutes, and thus form part of the immediate release part of the profile. According to the present invention, the proportion of the drug contained within the immediate release entity and dissolved within 30 minutes is at least 90%. And the proportion of the drug contained within the delayed release entity and released within 30 minutes is comprised between 0 and 20%, and preferably between 0 and 5%.

Among dosage forms able to match the requirement of a timed dual release profile and to comprise the two kinds of pharmaceutical entities defined above, the following may be cited: capsules, tablets, multilayer tablets, multicoated tablets.

The immediate release entity shall be understood in the present invention as a single pharmaceutical immediate release unit like for example an immediate release tablet or pellet, or several such units formulated into a capsule or a tablet ; as an immediate release matrix in a tablet ; as an immediate release layer, that can be incorporated in a multilayer tablet ; as an immediate release coating layer in a multicoated tablet or pellet.

The delayed release entity shall be understood in the present invention as a pharmaceutical delayed release unit such as, for example, a delayed release tablet or pellet, or several such units formulated into a capsule or a tablet ; as a delayed release core or a delayed release coating layer in a multicoated tablet ; as delayed release pellets within a disintegrating tablet.

Dosage forms where the immediate release entity and the delayed release entity are administered simultaneously but separately are also encompassed in the present invention.

The total amount of short acting hypnotic contained in the dosage forms according to the present invention depends on the individual drug.
For example, the dosage forms according to the invention typically contain from 10 to 30 mg of zaleplon or from 7.0 to 15 mg of zopiclone.
In the same way, the dosage forms according to the invention typically contain from 4 to 16 mg of zolpidem as zolpidem base, and preferably 6 to 12 mg of zolpidem as zolpidem base. The zolpidem may be incorporated as the base, or as a pharmaceutically acceptable salt of zolpidem. Among dosage forms comprising a salt of zolpidem rather than the zolpidem base, according to the invention, those comprising zolpidem hemitartrate are especially preferred.

In advantageous embodiments, dosage forms may be formulated in order to obtain a dissolution independent of the pH in the second release pulse. The preferred manner to achieve such a dissolution in the case of a basic short acting hypnotic, like zolpidem, zopiclone or zaleplon, is to add a pharmaceutically acceptable organic acid into the dosage form, according to methods known from one skilled in the art. Such dosage forms are preferred.
These pharmaceutically acceptable organic acids can be chosen for example among maleic, tartaric, malic, fumaric, lactic, citric, adipic or succinic acid and their acid salts where these exist, in the form of racemates or isomers, where these exist. According to the invention, acids particularly preferred are tartaric, fumaric, citric, and succinic and their acid salts.

Various formulations, not limiting the scope of the present invention, illustrating the invention are described hereafter:
(1) A mixture of immediate release and delayed release particles of dimension 0.2 - 2 mm, known variously as pellets, beads, granules or spheroids, within a capsule:
   The beads, pellets, granules or spheroids may be manufactured by any of the methods well known to one skilled in the art: granulation in a high speed granulator, extrusion followed by spheronisation, gradual coating of a sugar sphere with a mixture containing the drug etc.
   A part of the pellets, granules or spheroids is then coated for delayed release as described hereinafter. The coating must be impermeable to the drug on contact with aqueous fluid, but becomes permeable to the drug after a suitable period as described above, and not earlier, as a result either of erosion of the coating, or by increase in permeability of the coating, for example by the formation of aqueous pores, or by breakdown of the film, which may be obtained by means of:
   (i) a coating which contains one or more polymers impermeable to water and to drug molecules, such as ethylcellulose, ammonio methacrylate copolymer Type B, cellulose acetate, cellulose acetate butyrate, polyvinyl chloride, polyvinylacetate and one or more polymers which are permeable to water, such as hydroxypropylmethylcellulose, hydroxyethylcellulose, methylcellulose ammonio methacrylate copolymer Type A , the composition of the mixture being adjusted to allow gradual hydration of the film and a delayed release dissolution profile.
   (ii) a coating containing a mixture of polymers as in (i) which are physically incompatible with one another (immiscible). An example of such a mixture is that of ethylcellulose and methacrylate copolymers with quaternary ammonium groups (ammonio methacrylate copolymer Type A or B).
   (iii) a hydrophobic erodible coating, consisting of a wax such as carnauba wax, glyceryl behenate, or hydrogenated castor oil. This may be mixed with one or more insoluble diluants such as calcium dihydrogen phosphate or talc. It can be applied as the melted wax, for example in a fluid-bed coating apparatus.

   According to a preferred embodiment, in the case where one or more of the coating polymers is an ammonio methacrylate copolymer, a suitable cationic surfactant, or an amphoteric or zwitterionic surfactant is added. The surfactant diffuses into the coating, and at a given level provokes a sudden change in the films properties, provoking a sudden rapid release.
   This particular embodiment presents the advantage that the delayed pulse is accelerated and gives substantially more complete release of the active substance than for pellets, granules or spheroids coated with methacrylate copolymer without surfactants in their cores.
   Examples of such cationic surfactants are trimethyl-dimyristoyl-ammonium propane, dimethyl-dioctadecyl-ammonium bromide, trimethyl-cetyl-ammonium bromide (CTAB), dimethyl-didodecyl-ammonium bromide (DDAB(12)), benzalkonium chloride, cetylpyridinium chloride, cetramide.
   Examples of zwitterionic surfactants are the N-alkybetaines, the C-alkylbetaines, the N-alkylamidobetaines such as cocamidopropylbetain, the N-alkylglycines and the phosphatidylcholines or lecithines.
   This method is employed in the case of examples 2, 3 and 5.
   In the case of the coating being a hydrophobic wax, one or more non-ionic surfactants may be included in the formulation, in the core, in order to promote dissolution and erosion of the film.
   The core may contain other substances known to be necessary or advantageous to formation by one skilled in the art of pharmaceutical formulation, in particular an organic acid to maintain the pH at the interior of the pellet constant.
(2) A mixture of delayed release particles and an immediate release powder, within a capsule:
   The delayed release particles, known variously as granulates, pellets, beads, microspheres are those described above in (1). The immediate release powder is prepared by a simple mixture of the drug with pharmaceutically inactive substances, or by granulation of a mixture of drug mixed with pharmaceutically inactive substances, using one of the granulation methods well known to one skilled in the art of pharmaceutical formulation.
(3) A tablet containing delayed release coated pellets as described in (1) containing the drug imbedded in a matrix also containing the drug.
   Alternatively the tablet may consist of a mixture of delayed release coated pellets and of immediate release non-coated pellets containing the drug, imbedded in a matrix free from the drug.
   Alternatively the delayed release coated pellets may be furthermore coated with a layer containing the drug and other excipients allowing immediate release from that layer, imbedded in a matrix free from the drug.
   Alternatively the tablet may consist of one or more layers containing delayed release coated pellets containing the drug, imbedded in a matrix free from the drug and one or more layers containing the drug in an immediate release matrix.
   The matrix surrounding the pellets should preferably be formulated so that the compression into tablets does not interfere with the integrity of the membrane surrounding the pellets. On contact with fluid the tablet disintegrates, releasing the drug rapidly, from the matrix, or the immediate release pellets, or from the immediate release pellet coating, or from the immediate release layer, and then, after a set interval of time, releasing the drug from the delayed release pellets The pellet may be formulated with a pharmaceutically acceptable organic acid so as to maintain the micro-pH of the pellet during dissolution in the neutral pH conditions. The matrix consists of inert pharmaceutical substances such as well known to one skilled in the art of pharmaceutical formulation. In particular the matrix includes one or more diluants such as microcrystalline cellulose, lactose, mannitol, starch and one or more disintegrants, for example crospovidone, sodium starch glycolate and croscarmellose. Other excipients may also be included, lubricants, for example magnesium stearate, glyceryl stearate, and glyceryl behenate, binders, for example hydroxypropylmethylcellulose, ethylcellulose and povidone, glidants, for example talc and colloidal silicon dioxide.
(4) A capsule containing one or more immediate release tablets and one or more delayed release tablets. The immediate release tablet or tablets may be formulated by the methods well known to one skilled in the art. In addition to the drug they can contain inert pharmaceutical excipients, including one or more diluants, for example microcrystalline cellulose, lactose, mannitol, starch ; and may contain other excipients. These can include one or more binders, for example hydroxypropylmethylcellulose, ethylcellulose and povidone, lubricants, for example magnesium stearate, glyceryl stearate, and glyceryl behenate, disintegrants, for example crospovidone, sodium starch glycolate and croscarmellose, glidants, for example talc and colloidal silion dioxide.

The cores of the delayed release tablets may be prepared using the same excipients as the immediate release tablets except that additional substances may be added. In particular a pharmaceutically acceptable acid may be added to ensure liberation of the drug independent of the pH of the external medium.

The delayed release tablets are coated with a layer of polymer coating similar to those described for the multiparticulate pellet systems above. However some modification of the coating will be required because of the difference in surface area of the dosage form. It is usually necessary to apply a thicker coating on the tablet than on the pellets, and thus a higher proportion of water-permeable polymers will be required in the coating composition.
In the case of a hydrophobic wax coating, the wax may be mixed with a soluble diluant such as polyethylene glycol, and the mixture applied by press coating.
In the case of coatings containing a methacrylate copolymer, a cationic surfactant may advantageously be included within the delayed release tablet core. In the case of coatings containing a hydrophobic, waxy excipient such as carnauba wax or hydrogenated castor oil, a non-ionic surfactant may be included within the tablet core.

### List of figures:

Figure 1 shows an example of a *in vitro* timed dual release profile, where the immediate release pulse is 60% of the total amount of zolpidem, and the second pulse is 40%, starting at 90 minutes and finishing at 150 minutes.

Figure 2 shows an *in vitro* dissolution profile of the uncoated pellets containing zolpidem hemitartrate of example 1 at pH 2.

Figure 3 shows an *in vitro* dissolution profile of the coated pellets containing zolpidem hemitartrate of example 2 at pH 2 and 6.8.

Figure 4 shows an *in vitro* dissolution profile of the coated pellets containing zolpidem hemitartrate of example 3 at pH 2 and pH 6.8.

Figure 5 shows the *in vitro* dissolution profile of a capsule of example 4 containing a mixture of the uncoated pellets of example 1 and the coated delayed release pellets of example 3, containing 7.5 mg zolpidem hemitartrate in each type of pellet, at pH 2.

Figure 6 shows *in vitro* release profiles of coated pellets containing zolpidem hemitartrate of comparative example 1 at pH 2 and 6.8.

Figure 7 shows *in vitro* release profiles of coated pellets containing zolpidem hemitartrate of comparative example 2 at pH 2 and 6.8.

Figure 8 shows the *in vitro* dissolution profile of the coated pellets containing zolpidem tartrate of example 5.

The examples which follow illustrate the invention without limiting it:

### Example 1: Immediate release pellets containing zolpidem hemitartrate

1000 g of nonpareil beads 16/18 mesh were coated using a suspension with the following composition,

| | | |
|---|---|---|
| zolpidem hemitartrate | 11.54 % | 78.125 g |
| povidone K30 ¹ | 11.54 % | 78.125 g |
| ethanol | 76.92 % | 520.8 g |

| | | |
|---|---|---|
| ¹ Kollidon® commercialised by BASF | | |

The coating was carried out using a GPCG1 fluid bed coated-dryer (Glatt). The dissolution of the beads was measured using the method described in the European pharmacopoeia, with the rotating paddle apparatus, at a stirring speed of 50 rpm. Dissolution medium was 900 ml, 0.01M hydrochloric acid, at 37 ± 0.5°C. The amount of zolpidem hemitartrate dissolved was measured by UV spectrophotometry at 310 nm. The dissolution curve obtained is shown in figure 2.

### Example 2: Coated pellets:

Delayed release pellets containing zolpidem hemitartrate, tartaric acid and benzalkonium chloride as cationic surfactant

1000 g of nonpareil beads 16/18 mesh were coated using a suspension with the following composition,

| | | |
|---|---|---|
| tartaric acid | 6.0 % | 78 g |
| hydroxypropylmethylcellulose ¹ | 4.0 % | 53 g |
| benzalkonium chloride | 3.0 % | 39 g |
| purified water | 43.5 % | 567 g |
| isopropanol | 43.5 % | 567 g |

| | | |
|---|---|---|
| ¹ Pharmacoat® 603 commercialised by Shin-Etsu | | |

The pellets were then loaded with zolpidem hemitartrate by coating with the following solution, in a GPCG1 fluid bed coater-dryer:

| | | |
|---|---|---|
| zolpidem hemitartrate | 8.3 % | 78 g |
| povidone K30 ² | 8.3 % | 78 g |
| ethanol | 83.4 % | 784 g |

| | | |
|---|---|---|
| ² Kollidon® commercialised by BASF | | |

Finally the pellets were coated using a polymer solution of the following composition :

| | | |
|---|---|---|
| ammonio methacrylate copolymer Type B ³ | 11.40 % | 83.4 g |
| ammonio methacrylate copolymer Type A ⁴ | 0.93 % | 6.8 g |
| triethyl citrate ⁵ | 1.37 % | 10.0 g |
| isopropanol | 51.80 % | 379.0 g |
| acetone | 34.50 % | 252.0 g |

| | | |
|---|---|---|
| ³ Eudragit® RS100 commercialised by Röhm Pharma | | |
| ⁴ Eudragit® RL100 commercialised by Röhm Pharma | | |
| ⁵ Eudraflex® commercialised by Röhm Pharma | | |

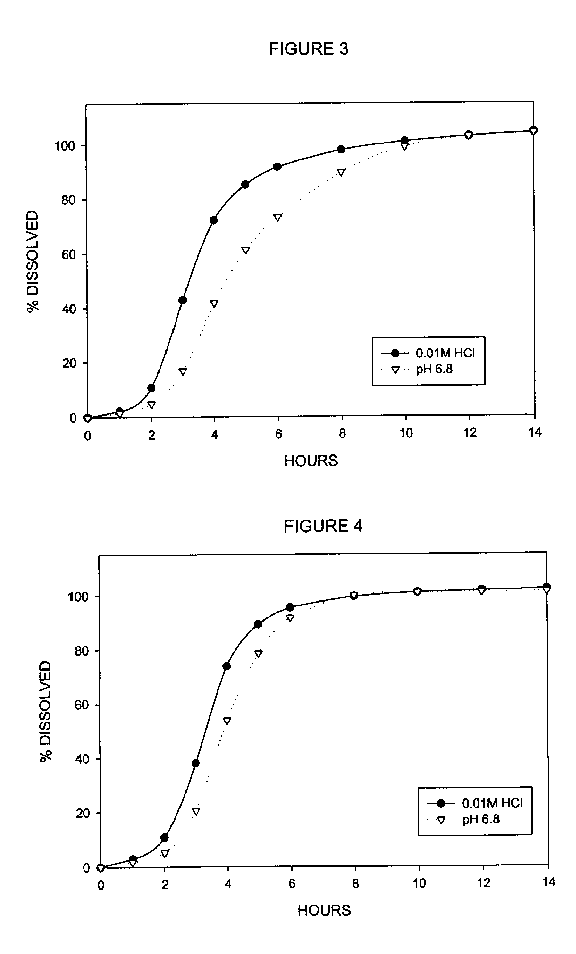

The dissolution profiles of the pellets were tested in 0.01M hydrochloric acid using the method described in example 1, and in a 0.02M pH 6.8 potassium phosphate buffer solution containing 0.1M sodium chloride, all other parameters being the same as for the test in hydrochloric acid. They are shown in figure 3.

### Example 3: Coated pellets:

Delayed release pellets containing zolpidem hemitartrate, tartaric acid and cetylpyridinium chloride as cationic surfactant

1000 g of nonpareil beads 16/18 mesh were coated using a suspension with the following composition,

| | | |
|---|---|---|
| tartaric acid | 6.0 % | 78.0 g |
| hydroxypropylmethylcellulose ¹ | 4.0 % | 53.0 g |
| cetylpyridinium chloride | 3.0 % | 39.0 g |
| triethyl citrate ² | 1.4 % | 18.2 g |
| purified water | 42.8 % | 557.0 g |
| isopropanol | 42.8 % | 557.0 g |

| | | |
|---|---|---|
| ¹ Pharmacoat® 603 commercialisé par Shin-Etsu | | |
| ² Eudraflex® commercialized by Röhm Pharma | | |

The beads were then loaded with zolpidem hemitartrate by coating, in a GPCG1 fluid bed coated-dryer, and finally coated using a polymer solution, using the same methods and compositions as described in example 2. The dissolution profiles of the pellets were measured as described in example 2. These are shown in figure 4.

### Example 4: capsule containing a mixture of immediate release and delayed release pellets containing zolpidem hemitartrate

Capsules containing 15 mg zolpidem hemitartrate were manufactured according to the following composition :

| Component | Mass per unit | Zolpidem hemitartrate content |
|---|---|---|
| uncoated beads of example 1 | 112 mg | 7.5 mg |
| coated beads of example 3 | 131 mg | 7.5 mg |
| hard gelatine capsule size 3 | | - |
| (Total) | | 15.0 mg |

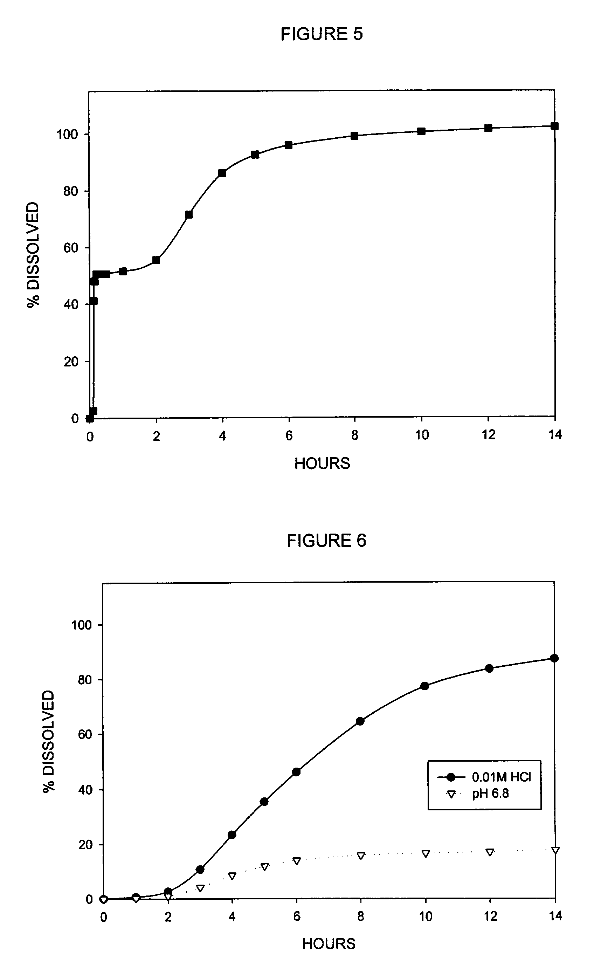

Their dissolution profile in 0.01M hydrochloric acid obtained as described in example 2, is shown in figure 5. The profile parameters in hydrochloric acid are : T₁ = 2.0 h ; T₂ = 5.0 h.

### Comparative example 1: coated pellets containing zolpidem hemitartrate

850 g of pellets coated with zolpidem hemitartrate of example 1 were coated in a GPCG1 fluid bed coater-dryer with the following solution

| | | |
|---|---|---|
| ammonio methacrylate copolymer Type B ¹ | 11.41 % | 129.6 g |
| ammonio methacrylate copolymer Type A ² | 0.92 % | 10.5 g |
| triethyl citrate ³ | 1.37 % | 15.6 g |
| isopropanol | 51.78 % | 588.0 g |
| acetone | 34.52 % | 392.0 g |

| | | |
|---|---|---|
| ¹ Eudragit® RS100 commercialised by Röhm Pharma | | |
| ² Eudragit® RL100 commercialised by Röhm Pharma | | |
| ³ Eudraflex® commercialised by Röhm Pharma | | |

After drying, in a ventilated oven at 35°C for 24 hours the dissolution profile of the pellets was measured in 0.01M hydrochloric acid and in pH 6.8, 0.02M phosphate buffer containing 0.1M sodium chloride as described in example 2. The profiles are shown in figure 6. Prolonged dissolution (over about 12 hours) was obtained at 0.01M hydrochloric acid, but the release rate was very slow at pH 6.8.

### Comparative example 2 : coated pellets containing zolpidem hemitartate and tartaric acid

735 g of nonpareil beads 16/18 mesh were coated using a suspension with the following composition:

| | | |
|---|---|---|
| tartaric acid | 10 % | 58.4 g |
| povidone K30 ¹ | 2 % | 11.5 g |
| ethanol 95% | 88 % | 505.0 g |

| | | |
|---|---|---|
| ¹ Kollidon® commercialized by BASF | | |

The coated pellets were then coated in a GPG1 fluid bed coater-dryer with the following solution,

| | | |
|---|---|---|
| zolpidem hemitartrate | 11.54 % | 78.125 g |
| povidone K30 ² | 11.54 % | 78.125 g |
| ethanol | 76.92 % | 520.8 g |

| | | |
|---|---|---|
| ² Kollidon® commercialized by BASF | | |

748 g of the zolpidem hemitartrate-tartaric acid-coated beads were then coated with the following solution.

| | | |
|---|---|---|
| ammonio methacrylate copolymer Type B ³ | 11.40 % | 62.5 g |
| ammonio methacrylate copolymer Type A ⁴ | 0.93 % | 5.1 g |
| triethyl citrate ⁵ | 1.37 % | 7.5 g |
| isopropanol | 51.80 % | 283.5 g |
| acetone | 34.50 % | 189.0 g |

| | | |
|---|---|---|
| ³ Eudragit® RS100 commercialised by Röhm Pharma | | |
| ⁴ Eudragit® RL100 commercialised by Röhm Pharma | | |
| ⁵ Eudraflex® commercialised by Röhm Pharma | | |

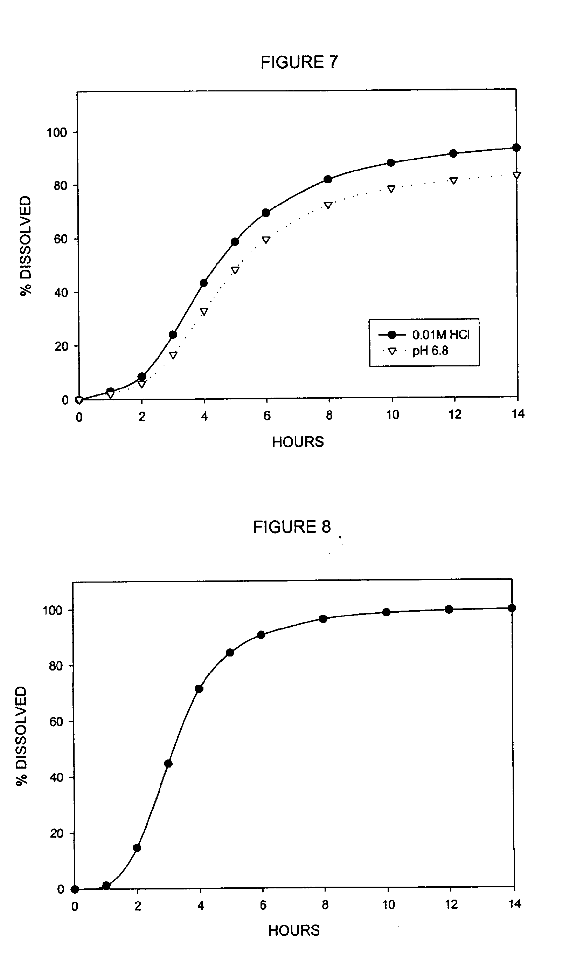

After drying, in a ventilated oven at 35°C for 24 hours the dissolution profile of the pellets were measured in 0.01M hydrochloric acid and in a pH 6.8 0.02M phosphate buffer containing 0.1M sodium chloride as described in example 2. The profiles are shown in figure 7. Dissolution was prolonged and independent of pH.
These two comparative examples show that the delayed release pellets comprising acid present a profile of dissolution independent of the pH and that the addition of a cationic surfactant to the tablet core increases the release rate and extent of release at both acid and neutral pH.

### Example 5 : Coated pellets :

Delayed release pellets containing zolpidem tartrate, tartaric acid, and cocamidopropylbetain as an amphoteric surfactant.

1000 g of nonpareil beads 16/18 mesh were coated using a suspension with the following composition, in a GPCG1 fluid bed coater-dryer:

| | | |
|---|---|---|
| tartaric acid | 5.0 % | 78.0 g |
| cocoamidopropylbetain ¹ | 2.5 % | 39.0 g |
| Povidone VA 64 ² | 5.0 % | 78.0 g |
| talc | 5.0 % | 78.0 g |
| purified water | 41.25 % | 643.5 g |
| ethanol 95° | 41.25 % | 643.5 g |

| | | |
|---|---|---|
| ¹ Amonyl® 380LC commercialized by Seppic | | |
| ² Kollidon® VA 64 commercialized by BASF | | |

The pellets were then loaded with zolpidem tartrate by coating with the following solution :

| | | |
|---|---|---|
| zolpidem tartrate | 8.3 % | 78 g |
| Povidone VA 64 ³ | 8.3 % | 78 g |
| ethanol 95° | 83.4 % | 784 g |

| | | |
|---|---|---|
| ³ Kollidon® VA 64 commercialized by BASF | | |

Finally 1000 g of the pellets were coated using a polymer solution of the following composition :

| | | |
|---|---|---|
| ammonio methacrylate copolymer Type B ⁴ | 11.40 % | 83.4 g |
| ammonio methacrylate copolymer Type A ⁵ | 0.93 % | 6.8 g |
| triethyl citrate ⁶ | 1.37 % | 10 g |
| isopropanol | 51.80 % | 379 g |
| acetone | 34.50 % | 252 g |

| | | |
|---|---|---|
| ⁴ Eudragit® RS100 commercialised by Röhm Pharma | | |
| ⁵ Eudragit® RL100 commercialised by Röhm Pharma | | |
| ⁶ Eudraflex® commercialised by Röhm Pharma | | |

After drying in a ventilated oven, at 30°C for 16 hours the dissolution profile of the pellets in 0.01 M hydrochloric acid was measured, using the method described in the European Pharmacopoeia, with the rotating paddle apparatus, at a stirring speed of 100 rpm. Dissolution medium was 900 ml, 0.01M hydrochloric acid at 37°C ± 0.5°C. The amount of zolpidem dissolved was measured by UV spectrophotometry at 310 nm. The dissolution curve obtained is shown in figure 8.

### Example 6 : Tablet containing coated delayed release pellets containing 6 mg zolpidem hemitartrate within a fast-disintegrating matrix containing 6.5 mg zolpidem hemitartrate.

Prolonged release coated pellets were manufactured as described in example 3. The pellets were then spray-coated using the same method with a layer of 20% by mass of microcrystalline cellulose. A granulate of the following composition was then prepared, by wet granulation:

| | |
|---|---|
| zolpidem hemitartrate | 3.0 % |
| lactose | 20.0 % |
| microcrystalline cellulose ¹ | 68.0 % |
| hydroxypropylmethylcellulose 606 | 3.0 % |
| crospovidone ² | 5.0 % |
| magnesium stearate | 1.0 % |

| | |
|---|---|
| ¹ Avicel®, commercialized by FMC | |
| ² Kollidon® CL, commercialised by BASF | |

Pellets and granulate were mixed and compressed into tablets using a rotary press. Each tablet contained 130 mg pellets and 217 mg of the granulate.

## Claims

1. A pharmaceutical composition comprising a short acting hypnotic or a salt thereof characterised in that it consists of a timed dual release dosage form adapted to release the short acting hypnotic over a predetermined time period, according to an *in vitro* profile of dissolution when measured in a rotating paddle apparatus of the European pharmacopoeia in aqueous buffer at 37°C, comprising two release pulses, the first being immediate and the second being delayed by a fixed time after the administration.

2. A pharmaceutical composition according to claim 1, characterised in that the first pulse has a maximum duration of 30 minutes.

3. A pharmaceutical composition according to claim 1 or 2, characterised in that the fixed time is between 50 and 200 minutes.

4. A pharmaceutical composition according to claim 3, characterised in that the fixed time is between 60 and 150 minutes.

5. A pharmaceutical composition according to any one of claims 1 to 4, characterised in that 40 to 70% of the total amount of the short acting hypnotic is released during the immediate release pulse.

6. A pharmaceutical composition according to any one of claims 1 to 5, characterised in that the delayed release pulse lasts between 30 and 200 minutes.

7. A pharmaceutical composition according to any one of claims 1 to 6, characterised in that the time for release of 85% of the total amount of the short acting hypnotic is between 2 and 6 hours.

8. A pharmaceutical composition comprising a short acting hypnotic or a salt thereof characterised in that it comprises two kinds of pharmaceutical entities: one immediate release entity and one delayed release entity.

9. A pharmaceutical composition according to claim 8, characterised in that it consists in a dosage form chosen among capsules, tablets, multilayer tablets, multicoated tablets.

10. A pharmaceutical composition according to claim 8 or 9, characterised in that it consists of a capsule comprising one or more immediate release tablets and one or more delayed release tablets.

11. A pharmaceutical composition according to claim 8 or 9, characterised in that it consists of a capsule comprising a mixture of delayed release particles and immediate release particles.

12. A pharmaceutical composition according to claim 8 or 9, characterised in that it consists of a capsule comprising a mixture of delayed release particles and an immediate release powder.

13. A pharmaceutical composition according to claim 8 or 9, characterised in that it consists of a tablet comprising a number of delayed release coated pellets comprising the drug imbedded in a matrix and alternatively in that
(i) the matrix comprises the drug,
(ii) immediate release non-coated pellets are mixed to the delayed release coated pellets,
(iii) the delayed coated pellets are further coated with a layer comprising the drug, allowing immediate release form that layer, imbedded in a matrix free from the drug,
(iv) the tablet consists of one or more layers comprising the delayed release pellets imbedded in a matrix free from the drug and one or more layers containing the drug in an immediate release matrix.

14. A pharmaceutical composition according to any one of claims 10 to 13, characterised in that the delayed release particles or tablets are coated with a mixture containing at least one ammonio methacrylate copolymer and the core contains a cationic surfactant.

15. A pharmaceutical composition according to any one of claims 10 to 13, characterised in that the delayed release particles or tablets are coated with a mixture containing at least one ammonio methacrylate copolymer and the core contains a zwitterionic surfactant.

16. A pharmaceutical composition according to claim 14, characterised in that the cationic surfactant is chosen among trimethyl-dimyristoyl-ammonium propionate, dimethyl-dioctadecyl-ammonium bromide, trimethyl-cetyl-ammonium bromide, dimethyl-didodecyl-ammonium bromide, benzalkonium chloride, cetylpyridinium chloride and cetrimide.

17. A pharmaceutical composition according to claim 15, characterised in that the zwitterionic surfactants are chosen among N-alkylbetaines, C-alkylbetaines, N-alkylamidobetaines, N-alkylglycines, phosphatidylcholines and lecithines.

18. A pharmaceutical composition according to claim 16, characterised in that the zwitterionic surfactant is cocamidopropylbetain.

19. A pharmaceutical composition according to claim 8, characterised in that the immediate release entity and the prolonged release entity are administered simultaneously but separately.

20. A pharmaceutical composition according to anyone of claims 1 to 18, characterised in that the prolonged release entity comprises a pharmaceutical acceptable organic acid which can be chosen among tartaric, malic, fumaric, lactic, citric, adipic or succinic acid and their acid salts, in the form of racemates or isomers.

21. A pharmaceutical composition according to any one of claims 1 to 20, characterised in that the short acting hypnotic belongs to the therapeutic classes of benzodiazepines, cyclopyrrolones, pyrazolopyrimidines, phenotiazines or imidazopyridines.

22. A pharmaceutical composition according to claim 21, characterised in that the short acting hypnotic is chosen among triazolam, temazepam, brotizolam, zolpiclone, (R)-zopiclone, zaleplon, alimemazine, zolpidem and their pharmaceutically acceptable salts thereof.

23. A pharmaceutical composition according to claim 21, characterised in that the short acting hypnotic is zolpidem or a pharmaceutically acceptable salt thereof.

24. A pharmaceutical composition according to claim 23, characterised in that the salt of zolpidem is zolpidem hemitartrate.
